# EUROPÄISCHE PATENTSCHRIFT

(11) **EP 1 790 379 B1**
(45) Veröffentlichungstag und Bekanntmachung des Hinweises auf die Patenterteilung: **05.01.2011**
(21) Anmeldenummer: 06024490.2
(22) Anmeldetag: 26.11.2006
(51) Int. Cl.: A61N 1/05

(54) **Retina Implantat zur elektrischen Stimulation der Netzhaut**
Retina implant for electrical stimulation of the retina
Implant rétinien destiné à stimuler la rétine

(30) Priorität: 28.11.2005 DE 102005056771
(43) Veröffentlichungstag der Anmeldung: 30.05.2007
(73) Patentinhaber: Gerding, Heinrich, 53359 Rheinbach (DE)
(72) Erfinder: Gerding, Heinrich, 53359 Rheinbach (DE)

(56) Entgegenhaltungen:
- WO-A-96/39221
- US-A- 5 935 155
- US-A1- 2004 054 407
- US-A1- 2004 078 064

## Beschreibung

### Bezeichnung der Erfindung

### Retina Implantat zur elektrischen Stimulation der Netzhaut

Die Erfindung betrifft eine Vorrichtung mit den Merkmalen des Oberbegriffs des Anspruchs 1, die als implantierbares Retina Implantat zur elektrischen Stimulation der Netzhaut des Auges Anwendung finden soll, bei dem die in das Augeninnere zu implantierenden Anteile sich auf penetrierende Elektroden beschränken, wobei der Elektrodenträger sich nicht im Auge befindet und die zur Stimulation verwendeten Elektroden die Lederhaut und Aderhaut des Auges oder Teile von beiden gemeinsam penetrieren und der Elektrodenträger sowie die elektronischen Bauteile zur Ansteuerung und Versorgung der Stimulationselektroden sich nicht im Augeninnern befinden. Der Erfindungsgegenstand soll vornehmlich medizinisch dazu verwendet werden, bei Patienten mit Störungen der Netzhautfunktion und einer daraus resultierenden Sehbehinderung wieder Wahrnehmungen zu erzeugen, die der Rehabilitation dienen. Weitere Funktionen des Netzhautstimulators können in Abhängigkeit von der Ausgestaltung von Stimulationsparametern die Beeinflussung des Funktionszustandes der Netzhaut mit dem Ziel einer Verbesserung der Wahmehmungsfunktionen, die Vermittlung von Informationen zum Tag-Nacht-Rhythmus sowie die Erzielung neurotropher und neuroprotektiver Effekte der Netzhaut und des Sehnerven sein. Gattungsgemässe Retina Implantate sind aus US 4,628,933 und US 5,935,155 bekannt.

### Erläuterung zum Stand der Technik

Es ist bekannt, dass durch elektrische Stimulation des Auges Wahrnehmungen hervorgerufen werden können, die allgemein als Phosphene bezeichnet werden (Übersicht bei Uhlig & Gerding, 2001). Es gilt nach § 3 Abs. 2 PatG als Stand der Technik, dass elektrische Stimulationen des Auges prinzipiell dazu genutzt werden können, bei Sehbehinderten oder Erblindeten, bei denen noch vitale Nervenzellen der Netzhaut (vor allem Ganglienzellen, aber auch vorgeschaltete neuronale Netzhautzellen) vorhanden sind, Phosphene zu erzeugen, die für die Orientierung der betroffenen Personen von Bedeutung sein können (weitere Fundstellenangaben unten). Auf dieser Grunderkenntnis bauen mehrere bekannte Lösungsansätze auf, die sich als wesentliche Merkmale in der Positionierung und technischen Ausführung der Stimulationselektroden, der Positionierung und technischen Ausführung der Elektrodenträger (Trägermaterial) und der Positionierung und technischen Ausführung der für den Betrieb der Elektroden erforderlichen elektronischen Bauelemente (Einzelbauelemente, integrierte Schaltkreise, Vorrichtungen zur Bereitstellung der Betriebsenergie des Systems) unterscheiden. Die bekannten Lösungsansätze können folgendermaßen eingeteilt werden:
A. Systeme mit intraokularer Positionierung der Elektroden und des Elektrodenträgers,
B. Systeme mit extraokularer Positionierung der Elektroden und des Elektrodenträgers ohne mechanische Durchdringung der Augenwand und ohne Penetration des Augeninneren.

In Kategorie A sind bekannt:
A1. Systeme mit subretinaler Positionierung von Elektroden und Elektrodenträgern. Diese unterteilen sich in:
   A1 a. Systeme, die als abgeschlossene Einheit ohne mechanisch fassbare Verbindung (elektrische Leiter, Brückenglieder) durch die äußere Augenhülle (Lederhaut) sich im Auge befinden und
   A1b. Systeme, die über eine Verbindung durch die Augenwand (Lederhaut) nach außen verfügen, wobei sich der Elektrodenträger und ein Teil der mit dem Elektrodenträger und den Elektroden verbundenen elektrischen/elektronischen Bauelementen und Schaltkreisen sich im Augeninnem befinden.
A2. Systeme mit epiretinaler Positionierung von Elektroden und Elektrodenträgern. Diese unterteilen sich in:
   A2a. Systeme, die als abgeschlossene Einheit ohne mechanisch erfassbare Verbindung (elektrische Leiter, Brückenglieder) durch die äußere Augenhülle (Lederhaut) sich im Auge befinden, wobei elektrische Bauelemente sich epiretinal oder in etwa in der Position der natürlichen Linse des Auges oder eines Kunstlinsenimplantates befinden und
   A2b. Systeme, die über Mikrokabel-/Leitungsverbindung durch die Augenwand (Lederhaut) nach außen verfügen, wobei sich ein Teil der mit dem Elektrodenträger oder den Elektroden verbundenen elektrischen/elektronischen Bauelementen und Schaltkreisen sich im Augeninnern befinden kann.

Die unter A und B beschriebenen Implantate sind beispielsweise bekannt aus: US 2,760,483, US-A-4628 933, DE 199 21 399 C2, DE 199 31 083 A1, US-A-4 628 933, WO 98/17343, US-A-5 935 155, US-A-6 647 297, US-A-6 324 429, US-A-5 935 155, EP 1 386 636 A3, EP 1 386 636 A2, US-A- 2004/01 27 957 A1, US 200 50 70 973, US 2004 193 232, JP 200 502 13 56, US 2005/000 4626 A1, US 2004/00 98 067, JP 200 402 4342, DE 103 04 831 A1, DE 103 29 615 A1, DE 197 05 988 C2.

Die dargestellten Systeme sind mit folgenden Nachteilen verbunden:

Die unter A subsumierten Systeme sind mit dem Nachteil verbunden, dass das Auge durch eine weite Schnittführung eröffnet werden muss und wesentliche Teile des Implantates (Elektroden und Elektrodenträger bei Version A1 b und A2b oder ein in sich abgeschlossenes System bei Version A1a und A2a) mit anhängenden Verbindungen und elektrischenlelektronischen Bauteilen/Schaftkreisen in das Augeninnere eingebracht, im Auge positioniert und befestigt werden müssen. Zum Zweck der Implantation ist es erforderlich, einen relativ umfänglichen und schwierigen operativen Eingriff durchzuführen mit der Notwendigkeit zu einer großen chirurgischen Öffnung des Auges, um entweder das in sich abgeschlossene gesamte intraokulare Implantat (A1a und A2a) oder Teile davon (Elektrodenträger und Elektroden, A1b und A2b) einbringen zu können. Zusätzlich zur Implantationsöffnung sind weitere Eröffnungen erforderlich, um den Glaskörper des Auges zu entfernen, Instrumente einbringen zu können, die der mechanischen Manipulation und Befestigung des Implantates im Auge und der Beleuchtung des Augeninneren während der Operation dienen. Zumindest Implantate vom Typ A1a machen es erforderlich, die noch vorhandene Linse des Auges oder ein ggf. schon vorhandenes künstliches Linsenimplantat zu entfernen. Bei einem Teil der Implantate vom Typ A1a wird der Kapselapparat der natürlichen Linse keine ausreichende Stabilisierung des vorderen Teils des Implantates gewährleisten. In diesen Fällen wird eine Befestigung des vorderen Anteils eines Implantats vom TypA1a durch transsklerale Nahttechniken (Stabilisierungsnähte) erforderlich sein.

Die für die oben genannten Implantate erforderlichen chirurgische Öffnungen und komplexen Eingriffe bergen zahlreiche Risiken wie z.B. eine Infektion, schwere Blutungen, erhebliche entzündliche Reaktionen des Auges, erhebliche Gefahr der Verletzung der Netzhaut, die Gefahr starker proliferativer Reaktionen des Auges, die Gefahr der Ausbildung von Membranen (zyklitische Membranen, retroiridale Membranen, retrocorneale Membranen) mit der Gefahr erheblicher unerwünschter Wirkungen auf den Gesamtzustand des Auges (Netzhautablösungen, Hypotonie und Schrumpfung des Auges (Atrophia bulbi, Phthisis bulbi), Trübungen, Sekundärglaukom, die Gefahr einer Wundinsuffizienz mit Undichtigkeit des Auges und die Gefahr von Implantatverlagerungen innerhalb des Auges. Ein Teil dieser unerwünschten Nebeneffekte kann andauernde erhebliche Beschwerden verursachen oder sogar zum Verlust des implantattragenden Auges führen. Da bei diesen Lösungen (alle unter A genannten, in besonderem Maße aber die unter A1b und A2b genannten) wesentliche Teile des Implantats oder das gesamte Implantat sich im Augeninnem (innerhalb der Begrenzung, die durch Lederhaut, Hornhaut und Aderhaut gebildet wird) befinden, kann es u.a. wegen der großen Fremdkörperoberftäche, die in Kontakt mit Gewebe des Augeninnem (Aderhaut, Pigmentepithel, Netzhaut, Iris, Ziliarkörper) sich befindet, zu starken Gewebereaktionen (Fremdkörperreaktionen, Membranbildungen, Ausbildung einer proliferativen Vitreoretinopathie, Ausbildung von retrokornealen oder zyklitischen Membranen kommen (Gerding et al, 2001), die die Funktion des Implantates und den Erhalt des Auges gefährden können. Weiterer Nachteil ist, dass bei epiretinalen und subretinalen Systemen wesentliche Teile des Implantates, wie Stimulationselektroden und Elektrodenträger, z.T. auch Verbindungsleitungen bzw. Verbindungsleitungsträger und weitere Komponenten des Implantats, direkt mit der Netzhautinnenfläche oder dem subretinalen Raum (Raum zwischen Aderhaut/retinalem Pigmentepithel und den äußeren Netzhautschichten) in Verbindung stehen. Dieses kann lokal zu unverwünschten sekundären Proliferationen der Netzhaut und der der Netzhaut benachbarten Kompartimente führen oder eine Degeneration der Netzhaut verursachen.

Epiretinale Implantate (Typ A2a und A2b) machen eine präzise und langzeitstabile Befestigung im Augeninneren erforderlich. Es ist bekannt, dass hierzu intrasklerale Nageltechniken (Nägel, "tacks", mikromechanische Befestigungselemente mit nagelähnlicher Verankerung in der Sklera) Verwendung finden können oder auch eine Klebung von Implantaten eine Befestigung bewirkt. Beide Verfahren können erhebliche Beschädigungen der Netzhaut zur Folge haben. Beschädigungen können durch den Befestigungsvorgang selbst ausgelöst werden oder durch Gewebereaktionen auf die Befestigungsstellen oder Befestigungsmedien. Darüber hinaus kann ein Dauerkontakt eines epiretinalen Implantates mit der inneren Netzhautschicht eine Degeneration der darunter liegenden Netzhaut zur Folge haben. Nachteilige Auswirkungen auf die Netzhautfunktion können durch Zellreaktionen gegenüber dem Implantat (Fremdkörperreaktionen, proliferative Reaktionen) entstehen, durch die die Funktion der kontaktierten Netzhaut beeinträchtigt werden oder vollständig verloren gehen kann.

Es ist bekannt, dass eine Explantation von Implantaten der genannten Ausführungen (A1 a, A1 b, A2a, A2b) sehr gravierende operative Eingriffe erforderlich macht, die isoliert von dem operativen Trauma der Implantation eigenständige irreversible Schädigungen des Auges bedingen kann (Walter & Mokwa, 2005).

Bei Implantaten vom Typ A1 b und A2b können Bewegungen des Auges und damit verbundene Verlagerungen der sich außerhalb des Auges befindlichen Implantatanteile auf das Augeninnere übertragen und eine Dislokation des im Auge befindlichen Anteils bewirken oder ggf. Schädigungen der Netzhaut des Sehnervenkopfes oder der Aderhaut bewirken.

Bekannte Systeme, bei denen ein Implantat angebracht werden soll, ohne das Auge zu eröffnen oder die Aderhaut des Auges zu penetrieren (Systeme vom Typ B) sind mit dem Nachteil verbunden, dass die zur Stimulation verwendeten Elektroden einen gemessen an der Größe der Nervenzellen und Nervenzellverbände der Retina großen Abstand aufweisen. Dieser Nachteil bringt es mit sich, dass Stimulationen nur mit einer geringeren Selektivität und Auflösung als bei direktem Netzhautkontakt möglich sind und dass zur Erzielung äquivalenter Stimulationseffekte höhere Stimulusparameter als bei einem direkten Kontakt der Netzhaut angelegt werden müssen. Vorschläge, die darauf abzielen, Elektrodenträger nach einer vorangehenden Totaleröffnung der Sklera direkt der Aderhaut aufzulegen, gehen mit einem sehr hohen Verletzungsrisiko der inneren Strukturen des Auges (Aderhaut/Netzhaut) einher.

Es wurde bislang nicht erkannt, dass zur medizinisch anwendbaren Stimulierung der Netzhaut im humanen Einsatz die Verwendung von Implantaten möglich ist, bei denen die verwendeten einzelnen Stimulationselektroden das Augeninnere durch Penetration der Augenwand oder der zuvor chirurgisch verdünnten Augenwand (Sklera) inklusive der Aderhaut des Auges erreichen. Behindert wurde dieser erfinderische Gedanke durch die Lehrmeinung, dass es nicht möglich sei, gefahrlos und ohne gravierende sekundäre Auswirkungen auf die Augenfunktion die Augenwand und die Aderhaut des Auges zu penetrieren. Insbesondere musste man nach gängiger Lehrmeinung vermuten, das dieses erhebliche Blutungen im Augeninnern, nachteilige Undichtigkeiten des Auges und erhebliche Sekundärreaktionen zur Folge haben würde. In eigenen, noch nicht veröffentlichten Versuchen an Tieraugen konnte gezeigt werden, dass die hier beschriebene Idee des Retina Implantats realisierbar ist, wobei die zahlreichen, z.T. oben erwähnten und befürchteten Gefahren für das Auge, die Augenfunktion und die Implantatfunktion sich nicht als real erwiesen.

### Literatur

Uhlig CE, Taneri S, Benner FP, Gerding H (2001) Electrical stimulation of the visual System. From empirical approaches to the development of visual Implants. Ophthalmologe 98:1089-1096

Gerding H, Uhlig CE, Taneri S, Busse H (2003) Die Implantation komplexer retinaler "ZWei-Insel-Prothesen" ist eine große chirurgische Herausforderung. Ophthamologe 100:(S1)211

Walter P, Mokwa W (2005) Epiretinale Sehprothesen. Ophtalmologe 102:933-940 Dokument US-A- 2004/054407 offenbart den nächstliegenden Stand der Technik.

### Aufgabe der Erfindung

Der Erfindung liegt die Aufgabe zugrunde, ein Retina Implantat zu schaffen, das die Nachteile bisheriger Implantate, insbesondere die Notwendigkeit eines großen und risikoreichen operativen Eingriffs mit weiter Eröffnung des Auges und großen Schnittführungen und Destabilisierungen im vorderen und/oder hinteren Augensegment und die Gefahr mechanischer Schädigungen des Augeninneren und ungünstiger proliferativer Sekundärreaktionen des Auges auf das Implantat weitgehend vermeidet, indem der Anteil an Bauelementen, die in das Auge eingebracht werden, weiter reduziert wird. Neben dem Verzicht auf eine Implantation von Empfängerelektronik, Spulen, weiteren elektronischen Schaltkreisen und Bauelementen (z.B. Kondensatoren), Verbindungsleitungen und anderen Verbindungselementen soll die Erfindung zusätzlich die Möglichkeit bieten, den Elektrodenträger für die Stimulationselektroden nicht in das Auge implantieren zu müssen. Neben einer Verminderung des chirurgischen Traumas und der postoperativen Gewebereaktion und möglichen Schädigungen von Geweben und Strukturen im Augeninnern soll das Retina Implantat darüber hinaus die Chance einer wenig traumatische Explantation ohne notwendige Eröffnung des Auges durch neuangelegte Schnitte oder Stichinzisionen ermöglichen. Die Erfindung soll nicht nur für die Übermittlung visueller Bildinformationen nutzbar sein, sondern auch andersartige Informationen und Effekte durch elektrische Stimulationen im Augeninneren auslösen können.

### Kennzeichnung der Erfindung

Erfindungsgemäß wird die Aufgabe von einem Retina Implantat mit den Merkmalen des Anspruch 1 gelöst.

Weil die für die Netzhautstimulation verwendeten Stimulationselektroden die Sklera oder zumindest die innenliegenden Schichten der Sklera und die Aderhaut bzw. die degenerierte Aderhaut gemeinsam penetrieren, ist es möglich, alle weiteren Bauelemente des Retina Implantats außerhalb des Auges anzubringen. Dies ermöglicht, eine Implantation vorzunehmen zu können, ohne das Auge eröffnen zu müssen mit der einzigen Ausnahme, dass die Stimulationselektroden und ggf. Referenzelektroden selbst die Augenwand penetrieren. Entsprechend der Ausführung werden direkte Schäden, die mit der Implantation verbunden sein können und solche, die aus den Folgen des Eingriffs als unerwünschte Gewebereaktion oder Gewebeschädigung resultieren und ebenso Sekundärreaktionen vermieden, die daraus resultieren, dass sich ein Implantat (mit Ausnahme der Stimulationselektroden) und damit eine wesentliche Fremdkörpermasse bzw. -oberfläche im Auge befindet. Des Weiteren kann eine Explantation oder ein Austausch des Implantats atraumatisch ohne das Auge eröffnende Schnitte oder Stichinzisionen vorgenommen werden. Weitere Vorteile bestehen darin, dass hochgradig ortsstabile und gleichzeitig atraumatische Befestigungsmethoden für den kritischen Intraokularraum nicht zur Anwendung gebracht werden müssen. Durch die externe Anbringungen aller Implantatanteile außer den Stimulationselektroden (und Referenzelektroden) unterliegen diese in der Größenauslegung, Formgebung und Oberflächenbeschaffenheit nicht den engen Limitierungen und Anforderungen, die eine intraokulare Implantation auferlegt.

In einer vorteilhaften Ausführung des Elektrodenträgers sind die einzelnen Stimulationselektroden nicht homogen, d.h. mit gleichem Abstand voneinander angebracht, sondern können im Abstand voneinander variieren, so dass unterschiedliche Dichteverteilungen der Stimulationselektroden auf dem Elektrodenträger resultieren. Die Anordnung der Elektroden mit unterschiedlicher Dichte auf der Fläche des Elektrodenträgers bietet den Vorteil, dass Netzhautstellen, je nach Anforderung mit einem unterschiedlich dichten Besatz an Stimulationselektroden versorgt werden können.

Bei einer vorteilhaften Weiterbildung wird die Länge der Stimulationselektroden unterschiedlich ausgelegt, so dass ein Teil der Elektroden bis in den Glaskörperraum hinein das Auge penetriert und andere Elektroden mit der Spitze bis in verschiedene Schichttiefen der Netzhaut und Aderhaut hineinreichen.

Vorteilhaft ist eine Weiterbildung bei der die freie, für die Stimulation verfügbare, nicht mit Isolierungsschichten versehene Oberfläche der Stimulationselektroden sich auf unterschiedliche Längenanteile gemessen von der Spitze der Stimulationselektrode erstreckt. Unterbrechungen der Isolierung der Stimulationselektroden in ihrem Verlauf in Form von Ringsegmenten oder Flächensegmenten sind ebenso von Vorteil. Die Variation der Elektrodenlänge und die Anbringung unterschiedlicher Verteilungen der Isolierung auf den Stimulationselektroden ist vorteilhaft für die Auslösung von Stimulationseffekten in verschiedenen Zellschichten und in verschiedenen Raumrichtungen der Netzhaut. Weitere Erläuterungen hierzu finden sich in der nachfolgenden Ausführungsbeschreibung.

In einer vorteilhaften Ausführung wird die Möglichkeit genutzt, Stimulationselektroden mit einer jeweils unterschiedlichen Gegenelektrode (Bezugselektrode, Referenzelektrode) oder mit verschiedenen Kombinationen aus mehreren Gegenelektroden (Bezugselektroden, Referenzelektroden) zur Erzielung eines Stimulationseffektes zu verschalten. Als Gegenelektrode kann dabei jede andere implantierte Stimulationselektrode einzeln oder in Kombination mit anderen Elektroden angesteuert werden. Eine für einen Stimulationseffekt als Gegenelektrode verwendete Stimulationselektrode wird hier und an anderen Stellen dieses Textes als Bezugselektrode bezeichnet. Als Gegenelektrode kann auch jede Referenzelektrode oder Kombination aus mehreren Referenzelektroden angesteuert werden. Als Referenzelektrode wird eine Elektrode bezeichnet, die ausschließlich als Gegenelektrode verwendet wird und nicht als aktive Stimulationselektrode vorgesehen ist. Die Kombination der jeweiligen Stimulationselektrode mit der oder den Gegenelektroden kann für jede Stimulation verändert werden.

Durch Kombination der Stimulationselektrode mit unterschiedlichen Gegenelektroden/Gegenelektrodenkombinationen werden um die Stimulationselektrode herum räumlich unterschiedliche elektrische Potentialverteilungen (Potentialfelder oder Feldpotentiale) generiert, die je nach Ausgestaltung unterschiedliche Nervenzellen oder Nervenzellgruppen der Netzhaut ansprechen. Auf diese Weise ist es möglich, mit einer einzelnen Stimulationselektrode unterschiedliche Nervenzellen und Nervenzellgruppen zu stimulieren, was in einer jeweils differenten Phosphenauslösung resultiert. Auf diese Weise ist eine einzelne Stimulationselektrode nicht nur für die Auslösung eines einzelnen Phosphens, sondern mehrerer Stimulationseffekte nutzbar.

Da die verschiedenen Nervenzellen der Netzhaut bekanntermaßen auch unterschiedliche raumzeitliche Schwellen für eine elektrische Stimulation aufweisen, wird über eine Variation der zeitliche Stimulusparameter und der Stimulusintensität eine weitere Gestaltungsmöglichkeit des Stimulationseffektes und damit der Phosphengestaltung genutzt. Vorteilhaft werden die genannten Effekte (Kombination von Stimulationselektrode und Gegenelektrode, Stimulusintensität und zeitliche Gestaltung des Stimulus) in Zusammenhang mit der varianten Elektrodenlänge und der unterschiedlichen Gestaltung der Isolierungsschichten der Elektroden genutzt. Auf diese Weise ist es möglich, mit einer einzelnen Stimulationselektrode weitere unterschiedliche Wahrnehmungen auszulösen. Die genannten Gestaltungsmöglichkeiten der Stimulation werden durch die externe Elektronikeinheit bestimmt. Welche Kombinationen des komplexen raumzeitlichen Arrangements sinnvolle Stimulationseffekte auslösen, wird vorteilhaft in einer Lemphase und einem Trainingsprogramm mit dem Implantatträger individuell ermittelt. Sinnvolle Kombinationen werden im Encoderprogramm abgespeichert und bei Bedarf während des Implantatbetriebs abgerufen.

In bevorzugten Weiterbildungen der Erfindung wird der Elektrodenträger so gestaltet, dass er nicht mehr eine fest zusammenhängende Einheit bildet, sondern Unterteilungen der festen Trägerstruktur aufweist (Multisegmentierung des Trägers) und in einer Weiterführung mehrere separate Elektrodenträger Verwendung finden. Jedes Segment des Trägers bzw. jeder Träger kann zwischen 1 und 100 Einzelelektroden tragen, wobei die Anzahl zwischen den einzelnen Segmenten variieren kann. Abstand und Verteilung der Elektroden der einzelnen Elektrodensegmente können unterschiedlich ausgelegt sein. Vorteile bietet eine Aufteilung des Elektrodenträgers für die Eintreibung der Elektroden im Rahmen des Implantationsvorgangs.

Wie oben ausgeführt, kann jeder der Stimulationselektroden als Gegenelektrode angesteuert werden und in Kombination mit einer für ein Stimulationsereignis angesteuerten Stimulationselektrode ein Paar bilden oder je nach jeweils angestrebtem Stimulationsziel in Kombination mit mehreren anderen Elektroden eine multiple (aus mehreren Elektroden gebildete Kombination) Gegenelektrode bilden. Neben dieser funktionell wechselnden Nutzung von Stimulationselektroden als Gegenelektrode ist es vorteilhaft, definitive Referenzelektroden anzubringen. Definitive Referenzelektrodenauslegungen (im Folgenden einfach als Referenzelektroden bezeichnet) sind: aa) Elektroden, die sich auf dem Elektrodenträger befinden und wie Stimulationselektroden die Augenwand penetrieren. Vorzugsweise werden solche Elektroden in einer längeren Bauform als Stimulationselektroden ausgelegt, so dass die Elektrodenspitze in den Glaskörper hineinragt. bb) Elektrodenflächen, die sich auf der Unterseite (zur Augenoberfläche gerichtete Implantatfläche) jedes Anteils des gesamten Implantates befinden können. cc) Gesonderte Referenzelektroden, die als elektrisch leitende Flächen mit der Ansteuerelektronik durch Zuleitungen verbunden sind, oder: dd) Penetrierende Elektroden, die räumlich getrennt von den Elektrodenträgem der Stimulationselektroden auf dem Auge befestigt werden. Durch das Ansprechen der Referenzelektroden und anderer Stimulationselektroden, die als Gegenelektroden dienen, kann in Zusammenhang mit den oben genannten Variationen der Stimulusparameter eine erhöhte Variabilität des Stimuluserfolges bewirkt werden, so dass mit einer jeweils angesprochenen Stimulationselektrode weitere unterscheidbare Reizerfolge erzielt werden.

Eine vorteilhafte weitere Ausführung der Empfangsspule besteht aus mehreren Teilspulen, die auf der Sklera nebeneinander oder überlappend angeordnet sind. Die Kombination mehrerer Einzelspulen kompensiert negative Auswirkungen von Augenbewegungen, die zu einer Verschiebung der Achslage von Sende- und Empfangsspule und damit zu einer verschlechterten Übertragung von Informations- und Energietransfer führen. Je nach Bedarf (Energiebedarf, Anzahl der Informationskanäle) werden einzelne Spulenelemente der Empfängerspule in Reihe oder parallel verschaltet. In einer weiteren vorteilhaften Ausführung werden einzelne Spulenelemente nur zum Informationstransfer oder zum Energietransfer genutzt.

Die Sendespule wird vorteilhaft in einer weiteren Ausführungsvariante ebenfalls aus mehreren neben- oder übereinander angeordnete Teilspulen bestehen, deren Ebenen auch gegeneinander gewinkelt angeordnet werden können. Sendspulen sind ebenfalls in Reihe oder parallel betreibbar. Einzelne Sendespulenelemente werden in einer weiteren Ausführungsvariante nur zum Transfer von Information oder von Energie auf das Implantat genutzt.

Eine weitere vorteilhafte Realisierung sieht die Nutzung der Informationsübertragung von der externen Elektronikeinheit auf das Implantat durch Licht (z.B. Infrarotlaserdiode) vor. Dies erweitert die Übertragungskapazität für lmplantatauslegungen mit größeren Anzahlen an Stimulationselektroden (über 50 Elektroden).

Für Systemauslegungen, bei denen eine höhere Anzahl an Stimulationselektroden vorgesehen sind, ist es vorteilhaft, wenn einzelne Elemente der Funktionskette Empfängerspule, nachgeschaltete elektrische und elektronische Bauelemente und Stimulationselektroden (Referenzelektroden) oder das gesamte System mehrfach angebracht und sinnvoll kombiniert werden.

Die durch das Retina Implantat elektrisch ausgelösten Stimulationseffekte an der Netzhaut können unterschiedlichen Zwecken dienen. Diese können sein: a) eine direkte Stimulation von Nervenzellen der Netzhaut mit dem Ziel der Auslösung von Wahrnehmungen, b) eine Stimulation von Nervenzellen oder Pigmentepithel der Netzhaut, mit dem Ziel, den Funktionszustand der Netzhaut zu beeinflussen, um erwünschte Änderung zu erzielen (Änderung des Adaptationszustandes, Empfindlichkeitsveränderungen der Netzhaut), c) durch Stimulation der Netzhaut Übertragung von Informationen zum Tag-Nacht-Rhythmus zu vermitteln, d) durch elektrische Stimulation die Auslösung neurotropher bzw. neuroprotektiver Effekte der Netzhaut zu bewirken.

Es ist möglich zum Nutzen des Implantatträgers andere Informationen als die einer Bildinformation auf das Implantat zu übertragen. So könnten codierte akustische Informationen, Informationen zur Wärmestrahlung und prinzipiell jede technisch durch Sensoren oder Messinstrumente erfassbare Messgröße in codierter Form auf die Elektroden übertragen werden.

### Spezieller Beschreibungsteil

Im Folgenden werden weitere Vorteile-und Merkmale der Erfindung durch bevorzugte Ausführungsbeispiele unter Bezugnahme auf die Zeichnungen beschrieben. Es zeigen:
**Fig. 1** einen sehr schematisierten Querschnitt durch ein Auge **18** mit einem Retina Implantat **1-8** und ebenfalls schematisiert die dazugehörigen externen Komponenten des Systems, die nicht implantiert werden **12-17, 31** und **32,** insgesamt als **19** bezeichnet.
**Fig**. 2a bis 2d schematisierte Querschnitte durch Augen **18** mit je einem Retina Implantat ähnlich **Fig.** 1 mit zusätzlich angebrachten Referenzelektroden **20, 20', 20"** und **20"'** in unterschiedlicher Ausführung.
**Fig.** 3a bis 3d Elektrodenträger ähnlich **Fig. 1****,** die zusätzlich mit Befestigungsösen **21** versehen sind und geteilt (**Fig.** 3b) bzw. mehrfach angelegt sind (**Fig.** 3d). Zusätzlich stellt **Fig.** 3c eine Deckplatte **33** dar.
**Fig. 4a** bis 4e Spitzen von Stimulationselektroden wie in **Fig.** 1 in unterschiedlicher Ausführung.
**Fig**. 5a bis 5c verschiedene beispielhafte Ausführungen der Empfangsspule **8** aus **Fig.** 1 einschließlich des Hüllkörpers **28** für die Empfangsspule(n).
**Fig**. 6a und 6b zwei vorteilhafte Ausführungen der Sendespule **14** aus **Fig**. 1 und des Hüllkörpers **31** der Sendespule(n).

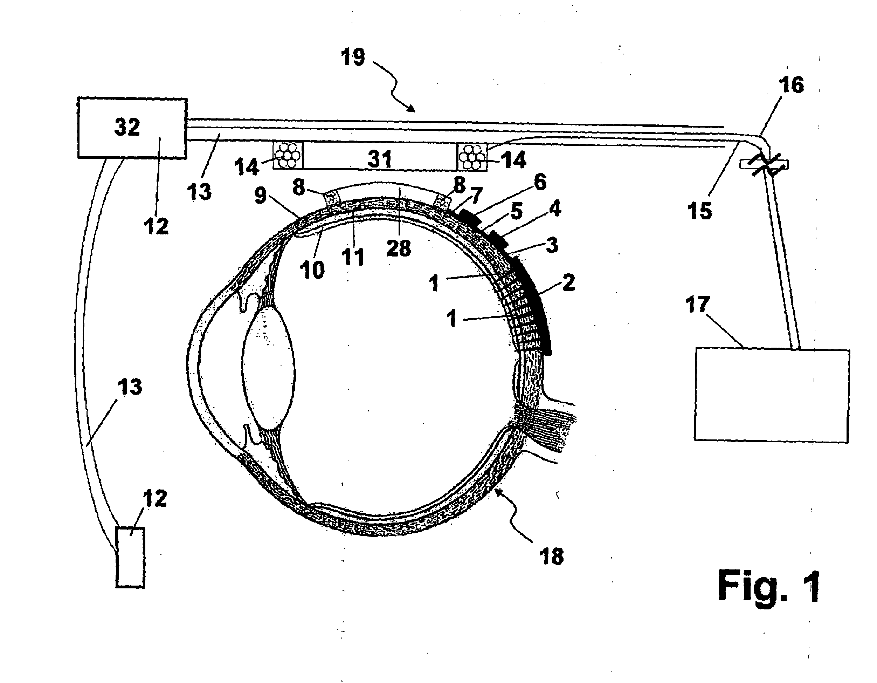

In **Fig.** 1 ist der Querschnitt durch ein Auge dargestellt, insgesamt als **18** bezeichnet. Am Auge markiert sind die schematisierte Lederhaut (Sklera) **9,** die Aderhaut (Chorioidea) **11,** und die Netzhaut (Retina) **10.** Das gesamte Retina Stimulator- System besteht aus zwei Haupteinheiten, dem Retina Stimulator (Implantat), das die mit **1, 2, 3, 4, 5, 6, 7, 8** und **28** bezeichneten Komponenten enthält und der externen Retina Stimulator Einheit (ERSE) mit den Komponenten **12, 13, 14, 15, 16, 17, 31** und **32,** insgesamt als **19** bezeichnet. Das Auge ist in Fig. 1 verbunden mit dem Retina Stimulator (Komponenten **1, 2, 3, 4, 5, 6, 7, 8** und **28**). Mit **1** sind die Elektroden (Stimulationselektroden) gekennzeichnet, die die Sklera oder Teile der Sklera und die Aderhaut bzw. die degenerierte Aderhaut penetrieren. Die Spitze der Elektroden kann in unterschiedliche Tiefe (bis in die Aderhaut, bis in die Netzhaut, bis in den Glaskörper hinein) reichen. Die Anzahl der Elektroden ist variabel (1-100 Stück pro Elektrodenträger oder Elektrodenträgeranteil). Bevorzugt wird eine Größenordnung von 10-25 Elektroden pro Träger. Die Stimulationselektroden werden aus elektrisch leitfähigen Materialien gefertigt (Gold, Platin, Platin-Iridium, Kupfer, Aluminium, Silizium, Stahllegierungen etc. und deren Legierungen) wobei die mit Gewebe oder Gewebsflüssigkeit in Kontakt befindlichen Flächen jeweils mit einem bioverträglichen Material zu versehen sind. Die Elektroden können aus einem Metall hergestellt werden. In vorteilhaften Ausführungen bestehen die Elektroden aus einem Kern (z.B. mechanisch beanspruchbares Kernmaterial) und einer darauf aufgebrachten äußeren Metallschicht. Der aus dem Elektrodenträger **2** herausragende Anteil der Elektroden **1** wird vorteilhaft teilweise von elektrisch isolierendem Material bedeckt sein (Kunststoffe, Oxydschichten, Polyoxydschichten). Die Elektroden sind nach vom zugespitzt, um den Eintriebvorgang zu erleichtern. Einzelne Elektroden weisen einen Durchmesser von 50 - 250 µm auf. Die Länge des aus dem Elektrodenträger hinausragenden Anteils der Stimulationselektroden variiert zwischen 300 und 1500 µm. Die Anordnung der Elektroden auf dem Elektrodenträger ist gleichmäßig verteilt oder in weiteren vorteilhaften Ausführungen in unterschiedlicher Dichte angebracht.

Die Elektroden **1** werden durch einen Elektrodenträger **2** auf bzw. in der Lederhaut fixiert. In einer ebenfalls vorteilhaften Ausführung befindet sich der Elektrodenträger nicht auf der äußeren Begrenzung der Sklera, sondern wird nach Teilabtragung oder vorübergehender Hochklappung eines an einer Kante noch mit dem Auge verbundenen Skleralappens in einer von außen betrachtet tieferen Schicht der Sklera aufgebracht. Bei Ausführungen, deren Implantation mit Hochklappen eines Skleralappens einhergehen, kann der Skleralappen benutzt werden, um die den Elektroden abgewandte Fläche des Elektrodenträgers wieder mit Gewebe zu bedecken. Dazu wird der Skleralappen zurückgelegt und durch Nähte mit der Sklera des Auges verbunden. Beim Elektrodenträger **2** handelt es sich um einen flachen Kunststoffkörper, der aus biologisch verträglichen Kunstoffmaterialien (z.B. Polyimid, Polymethylmetacrylat (PMMA), Silikon etc.) gefertigt wird. Der Außendurchmesser des Elektrodenträgers variiert zwischen 2 und 12 mm, sofern es sich um einen einzelnen soliden Elektrodenträger handelt.
Die Innenfläche des/der Elektrodenträger/s ist der Wölbung des Augapfels angepasst. In einer vorteilhaften Ausführung ist die der Sklera abgewandte Oberfläche aus einer Metallschicht gefertigt (bioverträgliches Edelmetall wie Gold, Platin, Titan und deren Legierungen).

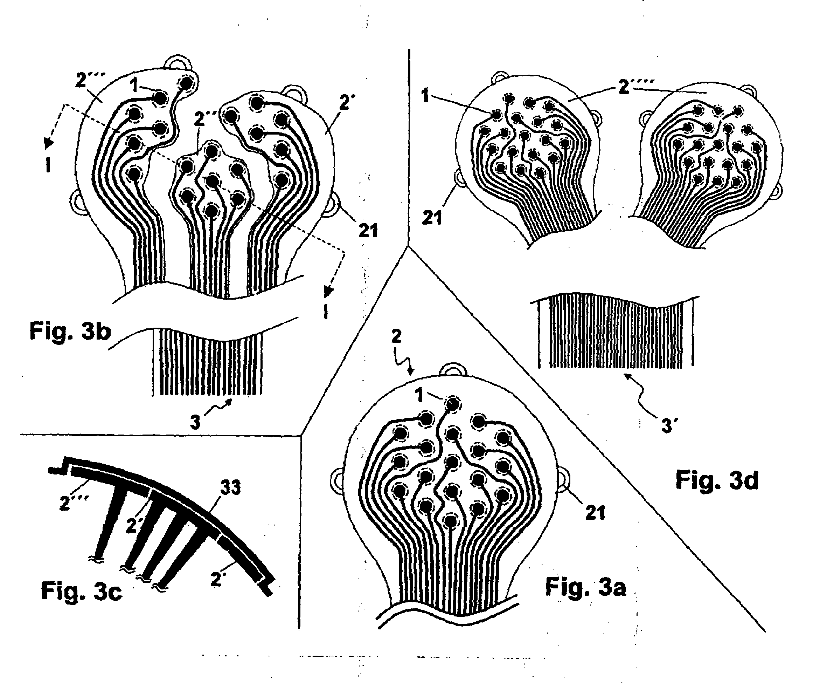

In einer weiteren vorteilhaften Ausführung (**Fig**. 3b) besteht der Elektrodenträger nicht nur aus einer einzelnen zusammenhängenden Fläche, sondern aus einer Anzahl unabhängig voneinander bewegbarer Einzelflächen (**2', 2", 2"'** in **Fig.** 3b), die jeweils mindestens eine Stimulationselektrode tragen. Dabei weist jede einzelne Fläche etwa einen Durchmesser von 1-10 mm auf. Ein Vorteil dieser Ausführung ist die erleichterte Penetration der Sklera, die nicht gleichzeitig mit allen Elektroden, sondern für jeden Elektrodenträger einzeln erwirkt werden muss.
Es werden am Elektrodenträger Ösen (**21** in **Fig.** 3a, 3b und 3c) angebracht, die der Befestigung durch intrasklerale Nähte dienen. Bei Elektrodenträgern, die aus einer Anzahl unabhängiger, gegeneinander beweglicher Einzelflächen bestehen, ist es vorteilhaft, die Einzelflächen nach der Implantation durch eine dünne Deckplatte (**33** in **Fig**. 3c) abzudecken, die mit Ösen versehen ist. Diese Ösen dienen der Nahtbefestigung der Deckplatte auf der Sklera. Die Deckplatte wird aus bioverträglichem Material gefertigt.

Der Elektrodenträger **2** in **Fig**. 1 beinhaltet Teile der elektrischen Zuleitungen **3**, die die Stimulationselektroden mit vorgeschalteten elektronischen Schaltelementen **4** (in **Fig**. 1 verbinden. Die Zuleitungen bestehen aus Material, das typischerweise für elektrische Leiter verwendet wird (Kupfer, Gold, Platin, Iridium, Titan, Aluminium oder deren Legierungen). In einer bevorzugten Ausführung bestehen die Zuleitungen aus Leiterbahnen, die durch einen photolithographischen Prozess hergestellt werden und sich auf einem Träger aus z.B. aus Polyimid befinden. Die Stimulationselektroden können direkt mit diesen Zuleitungen verbunden werden (Bonding, Verschmelzungstechnik, elektrisch leitende Klebung) oder in einer anderen vorteilhaften Ausfertigung durch Kontaktzungen, die von den Stimulationselektroden ausgehen, mit den Zuleitungen verbunden sein (durch Bonding, Verschmelzung, elektrisch leitende Klebung). In einer weiteren vorteilhaften Ausfertigung befindet sich auf der Rückseite (die dem Auge abgewandten Seite) der Zuleitungen und der Stimulationselektroden eine solide Stützfläche. Diese Stützfläche besteht aus Glas oder Kunststoffen und dient der mechanischen Stabilisierung der Zuleitungen und der Elektroden insbesondere in Hinblick auf die bei der Implantation auftretenden Kräfte, die zum Eintreiben der Elektroden in das Auge erforderlich sind.

Der Elektrodenträger **2** in **Fig**. 1 ist durch einen Leiterbahnenabschnitt **3** mit der Funktionseinheit A (FEA) **4** verbunden. Diese Verbindung ist vorzugsweise bezogen auf die Längsachse des Implantates flexibel ausgelegt, so dass der Implantatverlauf leicht der Wölbung des Auges angepasst werden kann. Es ist von Vorteil, den Leiterbahnenabschnitt **3** in einem auf der Oberfläche meandierenden Verlauf zu fertigen, um individuelle Lageanpassungen bei der Implantation besser berücksichtigen zu können. Der Leiterbahnenabschnitt **3** enthält eine Vielzahl einzelner elektrischer Leiter, die gleich oder unterschiedlich in der Bemessung ausgelegt sein können. Er besteht aus einer Elektrodenträgerschicht, die z.B. aus Polyimid, Epoxydharz oder PMMA gefertigt werden kann. Die elektrischen Leiterbahnen bestehen aus elektrisch leitfähigen Metallen wie z.B. Gold, Titan, Platin, Iridium, Aluminium oder Kupfer (oder deren Legierungen). Fertigungstechniken hierfür (Aufdampfen oder Sputtern von Metall auf den Träger, photolithographische Mikrostrukturierung) entsprechen dem Stand der Technik. Die Leiterbahnen sind zur elektrischen Isolierung (Einkapselung) gegenüber dem Urngebungsmilieu in Kunststoffmaterial eingekapselt (Hüllmaterial). Bevorzugt handelt es sich dabei um Material, das dem der Elektrodenträgerschicht entspricht. Es kann vorteilhaft sein, die Leiterbahnen vor der Einkapselung mit haftvermittelnden oder passivierenden chemischen Verbindungen zu beschichten. Zur Verbindungsherstellung der Leiterbahnen mit der FEA und den Stimulationselektroden eignen sich Bonding-Techniken, Feinlöttechniken oder Verfahren des leitfähigen Klebens.

Die Funktionseinheit A (FEA) **4** beinhaltet die für die Ansteuerung der Stimulationselektroden **2** erforderlichen elektronischen Bauelemente sowie elektronische Bauelemente bzw. integrierte Schaltkreise, die die Decodier- und Demultiplexing-Funktion für die Ansteuerung der einzelnen Stimulationselektroden bereitstellen. Die elektronischen Bauelemente bzw. Schaltkreise der Funktionseinheit A **4** werden durch Kunststoff umhüllt, der vorzugsweise dem Hüllmaterial des Leiterbahnenabschnitts **3** entspricht. Auch hier kann es vorteilhaft sein, die Bauelemente und Schaltkreise vor Anbringung des Hüllmaterials mit haftvermittelnden oder passivierenden chemischen Verbindungen zu beschichten.

Die Funktionseinheit A (FEA) **4** in **Fig.** 1 und Funktionseinheit B (FEB) **6** sind durch einen Leiterbahnenabschnitt **5** verbunden. Für Fertigung und Ausführung von **5** gelten anlog die Beschreibungen zum vorbeschriebenen Leiterbahnenabschnitt **3,** wobei die Anzahl und Ausführung der elektrischen Leitungen von den des Leiterbahnenabschnitts **3** abweichen kann. Die Funktionseinheit B **6** beinhaltet elektrische und elektronische Bauelemente und integrierte Schaltungen, die folgenden Aufgaben dienen: Wandlung der mit der Empfängerspule **8** aufgenommenen elektromagnetischen Energie (Wechselspannung zu Gleichspannung), Glättung und Regelung der Gleichspannung, Komponenten zur Dekodierung der Ober elektromagnetische Koppelung übertragenen Informationen und Umsetzung der dekodierten Informationsinhalte in Signale an die Multiplexer- und Stimulationselektronik. In weiteren Realisierungen kann es von Vorteil sein, elektrische Komponenten der Funktionseinheit B **6** wahlweise mit der Empfängerspule **8** oder/und der Funktionseinheit A **4** in einer gemeinsamen Einheit zusammenzufassen. Anteile der Funktionseinheit A **4** und B **6** können auch in den Elektrodenträger **2** integriert werden. Die Umhüllung von Funktionseinheit B **6** erfolgt analog zur Funktionseinheit A **4**. Ein weiterer Leiterbahnenabschnitt **7** verbindet die Funktionseinheit B **6** mit der Empfangsspule **8**. Die Ausführung des Leiterbahnenabschnittes **7** entspricht derjenigen des Leiterbahnenabschnittes **3**, wobei die Anzahl an darin enthaltenen Verbindungsleitungen und deren Bemaßung davon abweichen kann. Die Hüllkörper der Funktionseinheiten A **4** und B **6** sind seitlich mit Oesen versehen, die zur Nahtbefestigung auf der Sklera dienen. Die Empfangsspule **8** dient der induktiven Aufnahme elektromagnetischer Energie, die zum Betrieb der Funktionseinheiten A **3** und B **6** und der Stimulationselektroden **2** benötigt wird. Darüber hinaus dient die Empfangsspule **8** dem induktiven Empfang codierter Informationsinhalte. Die Empfangsspule **8** wird aus einem Metall hergestellt, das für Spulendrähte oder elektrische Leitungen Verwendung finden kann (Gold, Kupfer, Platin, Iridium, Titan, Aluminium oder deren Legierungen). Die Spule kann als konventionelle Wickelung von Draht oder auch als Leiterbahnenanordnung auf einem Trägermaterial (z.B. Polyimid) hergestellt werden. Die Anzahl an Windungen kann zwischen 5 und 100 variieren. Das Spulenmaterial ist, wie die elektrischen Leiter der Leiterbahnenträger **3, 5, 7**, von Hüllmaterial umgeben, vorzugsweise demselben Material wie bei den Leiterbahnenträgem und kann ebenso durch haftvermittelnde bzw. passivierende Verbindungen vor der Einhüllung beschichtet werden. Der Hüllkörper **28** kann den von den Spulenwindungen geformten Innenraum aussparen oder in einer anderen vorteilhaften Ausführung teilweise oder komplett ausfüllen. Eine weitere vorteilhafte Ausführung enthält in diesem Innenraum einen Magnetkern, der aus allgemein für Magnetspulen verwendetem Material (z.B. Ferrit) besteht. Ein vorhandener Magnetkern wird ebenfalls vollständig vom Hüllkörper umgeben. Die elektrischen Verbindungen zwischen Empfangsspule und Leiterbahnen des Leiterbahnenträgers **7** werden, ebenso wie die zwischen allen Leitern der Leiterbahnenträger **3**, **5** und den Funktionseinheiten A **4** und B **6**, durch Bonding, Verschmelzung oder elektrisch leitende Klebungen hergestellt. Der Hüllkörper der Empfangsspule weist außen vorzugsweise bis zu 6 Ösen **21** (in **Fig.** 3a, 3b und 3c) auf, die der Nahtbefestigung mit nicht resorbierbaren Nahtmaterialien auf der Sklera dienen.

Die externe Retina Stimulator Einheit (ERSE) **19** mit den Komponenten **12, 13, 14, 15, 16, 17, 31** und **32** befindet sich außerhalb des menschlichen Körpers und überträgt Informationsinhalte und Betriebsenergie drahtlos durch elektromagnetische Koppelung (Induktion) auf den Retina Stimulator (implantat).

Die ERSE besteht aus einem Träger **12,** der weitgehend wie ein übliches Brillengestell konfiguriert ist. In dem Träger **12** kann in üblicher Weise ein Brillenglas **13** zur optischen Korrektur oder auch zum Blendschutz oder der Maskierung des Auges durch getönte Gläser eingearbeitet werden. Dies kann zur optischen Korrektur bei noch vorhandenen Sehresten, zur Vorbeugung von Blendungseffekten bei Restfunktion oder zu kosmetischen Effekten genutzt werden. Vorzugsweise im Träger **12** (in Position **32**) oder - je nach gewünschter Tragerausführung - am Träger ist die Bildaufnahmeeinheit (Kamera, z.B. CCD- oder CMOS-Technologie) untergebracht. Über Verbindungsleitungen **16,** die im Bügel **13** (ähnlich einem Brillenbügel) des Trägers integriert oder an diesem angebracht sind, wird die Bildaufnahmeeinheit mit Betriebsenergie versorgt. Weiterhin sind mit **16** alle Verbindungsleitungen bezeichnet, die zwischen der externen Elektronikeinheit (EEE) **17** und der Kamera (in **12,** Position **32**) verlaufen und dem Betrieb der Kamera und dem Informationstransfer dienen. Die externe Elektronikeinheit **17** (nicht maßstabsgerecht wiedergegeben in **Fig**. 1), beinhaltet a) eine Energiebereitstellungseinheit (Batterien, Akkumulatoren, Brennstoffzellen) b) eine Vorrichtung zur Ladung derselben c) eine Pulssignalwandlereinheit für die Verarbeitung aufgenommener Bildinhalte, d) einen Encoder, der der Umsetzung der Bildinformationen in adäquate, codierte lmpulsfolgen dient, e) eine Sendeeinheit, die die Sendespule **14** elektrisch betreibt. Der in **17** enthaltene Encoder dient der Umsetzung von Bildinformation in zeitliche lmpulsfolgen und Impulsformen, die den Bedürfnissen nach Informationscodierung der jeweils kontaktierten Nervenzellen der Netzhaut entsprechen. Der Encoder ist modulierbar und kann Über eine Steckverbindung oder, in anderen vorteilhaften Ausführungen, drahtlos (Infrarot, elektromagnetisch) in seinem Übertragungs- und Encodierverhatten beeinflusst werden. Über Verbindungsleitungen **15,** die wahlweise wieder im oder am brillenbügelähnlichen Abschnitt **13** des Trägers **12** verlaufen, wird die Sendespule **14**, die ebenfalls am Träger **12** befestigt ist, elektrisch betrieben. Die Sendespule **14** ist so angebracht, dass die mittenzentrierten Querachsen von **14** und von der Empfängerspule **12** sich bei Geradeausblick möglichst treffen. Die Sendespule **14** hat einen Durchmesser von etwa 5 bis 40 Millimeter und enthält ca. 10 bis 250 Windungen. Sie ist gefertigt aus üblichem Material für elektrische Spulen (vorzugsweise Kupfer oder Aluminium).

Die Sendespule kann einen Magnetkern (z.B. Ferrit) enthalten. Sie ist zweckmäßigerweise mit einer Kunststoffkapsel versehen und kann nach Formgebung wie der Seitenschutz eines Brillengestells gestaltet werden. In weiteren vorteilhaften Ausführungen besteht die Sendespule aus mehreren einzelnen, unabhängig oder kombinatorisch angesteuerten Spulen, die räumlich nebeneinander oder überlappend angeordnet sein können.

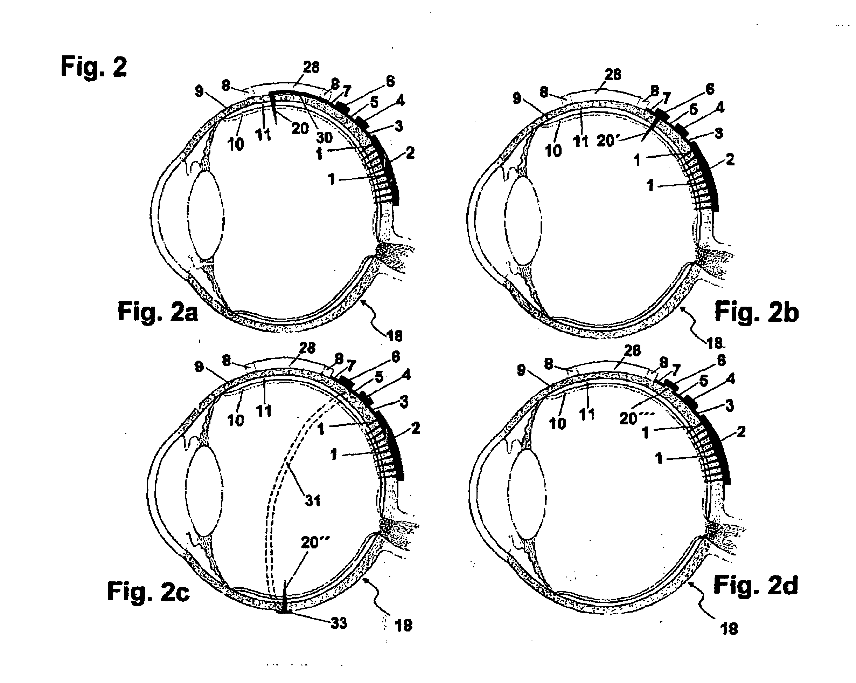

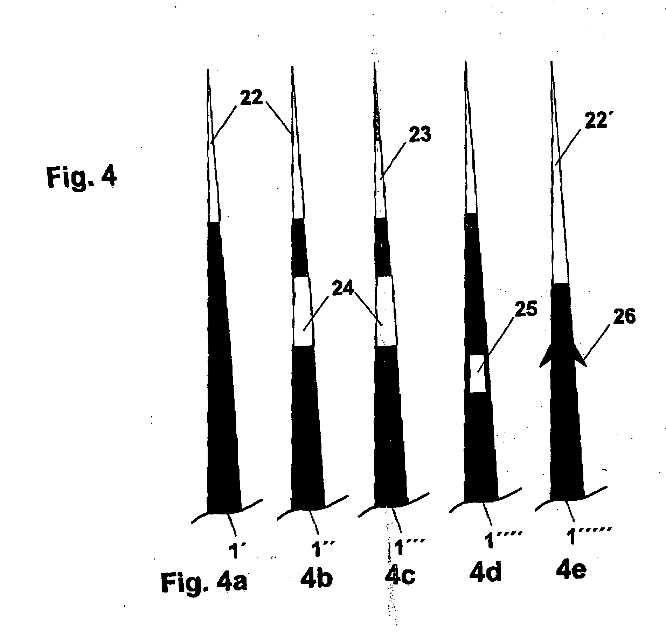

In **Fig**. 2 a bis d sind weitere Realisierungen des Retina Implantats aus **Fig**. 1 veranschaulicht, die mit zusätzlichen Referenzelektroden bestückt sind. In **Fig.** 2a ist eine mit dem Hüllkörper **28** verbundene penetrierende Referenzelektrode **20** skizziert, die über die Verbindungsleitung **30** angesteuert wird. In **Fig**. 2b ist eine penetrierende Referenzelektrode **20'** mit der Funktionseinheit B **6** verbunden. Eine Ausführung mit separater penetrierender Referenzelektrode **20",** die über die Zuleitung **34** mit der Funktionseinheit A 4 verbunden ist und über einen separaten Elektrodenträger **33** verfügt, ist in **Fig.** 2c schematisiert. **Fig.** 2d stellt ein Beispiel mit einer flächigen Referenzelektrode **20"'** auf der skleraseitigen Oberfläche der Funktionseinheit B **6** dar. Die in **Fig.** 2 a bis d dargestellten Referenzelektroden **20**, **20', 20"** oder **20"'** werden einzeln oder in vorteilhaften Weiterführungen multipel angebracht. Anbringungsort für diese einzelnen oder multiplen Elektroden kann jede Implantatkomponente, z.B. **2, 3, 4, 5, 6, 7, 8, 28, 33** und/oder **34**, oder deren modifizierte Ausführung, sein. Die verschiedene Ausführungen der Stimulationselektroden, z.B. **1, 1', 1", 1"', 1"", 1""'** (**Fig.** 1 und **Fig.** 4 a bis e) und der Referenzelektroden **20, 20', 20", 20"'** (**Fig.** 2a-d) sind beliebig kombinierbar.

**Fig**. 3 a bis c veranschaulicht vorteilhafte Ausführungsformen des Elektrodenträgers **2** aus **Fig. 1****.** In **Fig. 3a** ist der Elektrodenträger **2** mit drei Ösen **21** zur Befestigung auf der Sklera versehen. **Fig.** 3b skizziert die Ausführung eines geteilten Elektrodenträges, der aus den Komponenten **2', 2"** und **2"'** besteht und mit einem gemeinsamen Leiterbahnenträger **3** verbunden ist. Die Anteile **2'** und **2"** weisen Ösen **21** zur Befestigung auf der Sklera auf. Vorteilhaft für die Befestigung eines geteilten Elektrodenträgers nach dem Aufbau von **Fig**. 3b ist die zusätzliche Anbringung einer Stabilisatorplatte **33**, die in **Fig**. 3c beispielhaft skizziert ist (Querschnitt entsprechend der Markierung I aus **Fig**. 3b). In **Fig**. 3d ist eine Variante mit mehreren Elektrodenträgen **2""**, die durch einen gemeinsamen Leiterträger **3'** verbunden sind, wiedergeben.

**Fig. 4** gibt Ausführungsbeispiele von Stimulationselektroden **1** bis **1""'** an. Helle Flächen markieren Anteile der Stimulationselektroden ohne elektrische Isolierschicht, dunkel sind Bereiche mit elektrischer Isolierschicht gekennzeichnet. **Fig.** 4a zeigt eine Stimulationselektrode mit isolationsfreier Spitze **22,** **Fig**. 4b ein Modell mit isolationsfreier Spitze und ringförmigem Bereich **24** ohne Isolierung. **Fig.** 4c zeigt eine Stimulationselektrode **1"'** mit zwei ringförmigen isolierungsfreien Flächenanteilen. Das Modell **1""** in **Fig.** 4d, eine Modifikation von **1',** weist neben der isolatorfreien Spitze eine Fensterung **25** der Isolation auf. Die isolationsfreie Spitze kann in der Länge unterschiedlich ausgelegt werden, wie in **Fig.** 4e mit **22** gekennzeichnet. In diesem Beispiel ist die zusätzliche Ausstattung mit Widerhaken **26** dargestellt. Widerhaken dienen der Sicherung und Stabilisierung der Stimulatorelektroden in der Sklera und tragen zur mechanischen Stabilisierung des Gesamtimplantats bei. Die in den **Fig.** 4a bis 4e skizzierten Ausführungsmerkmale der Elektroden sind für verschiedene Elektrodenmodelle kombinierbar. Die Ausführungsvorschläge gelten analog für die Fertigung penetrierender Referenzelektroden wie in den Beispielen **20, 20', 20", 20"'** der **Fig**. 2a bis 2c dargestellt.

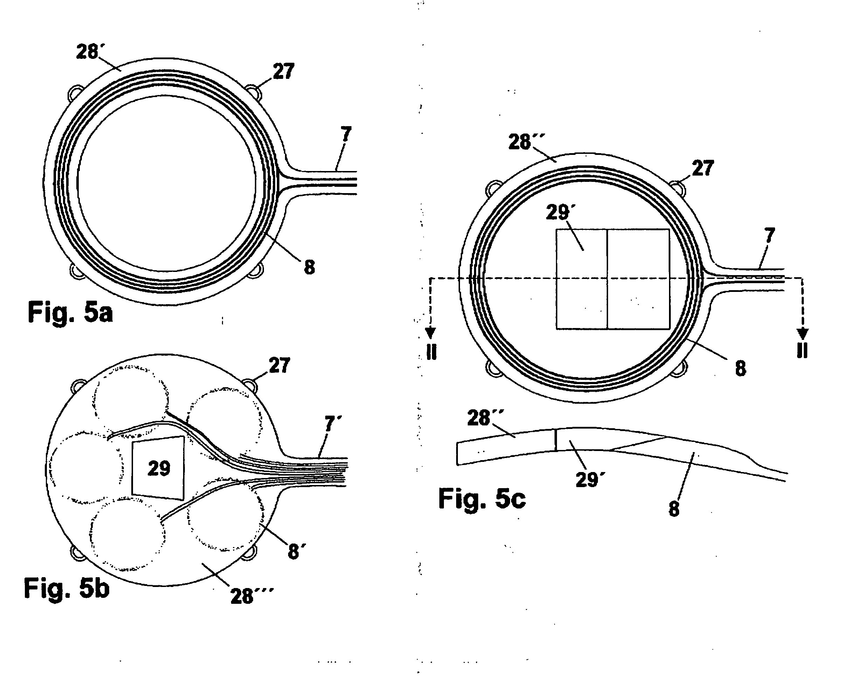

Vorteilhafte Ausführungen der Empfängerspule **8** aus **Fig.** 1 sind in **Fig**. 5a bis 5c schemenhaft dargestellt. **Fig**. 5a zeigt eine Ausführung mit einem ringförmig gewölbten Hüllkörper **28',** an dem vier Ösen **27** angebracht sind, die der Befestigung auf der Sklera dienen. **Fig.** 5b stellt eine Ausführungsvariante mit mehreren kleineren einzelnen Empfangsspulen **8'** und einem fast geschlossenen Hüllkörper **28"** dar. Zentral ist eine Aussparung **29** vorgesehen, die der Durchführung eines Augenmuskels, bevorzugt des Musculus rectus lateralis, dient. **Fig**. 5c stellt eine Ausführung, ähnlich derjenigen in **Fig**. 5a dar, bei der der Hüllkörper als gewölbte Fläche **28"** gestaltet ist. Die Aussparung **29'** dient der Durchführung eines Augenmuskels (vorzugsweise Musculus rectus lateralis). Zur besseren Führung des Muskels ist die Aussparung **29'** in der hinteren Begrenzungskante schräg verlaufend ausgelegt. Ein Querschnitt (markiert mit II in **Fig**. 5c) ist unten in **Fig**. 5c dargestellt.

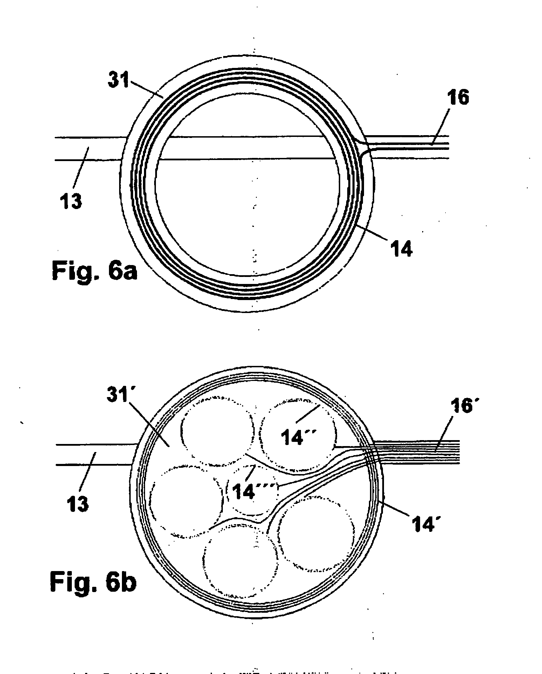

Ein Teilausschnitt des Trägers **13** mit der Sendespule **14** aus **Fig.** 1 ist in **Fig.** 6a herausgestellt. In **Fig.** 6a ist die Sendespule **14** von einem ringförmigen Hüllkörper **31** umgeben. **Fig.** 6b skizziert eine Ausführungsvariante mit Sendespulen **14', 14", 14"'**, die in unterschiedlichem Durchmesser gefertigt sind. Zur Vereinfachung sind in dieser Skizze mehrere Leitungsverbindungen zu den Spulen jeweils durch einfache Linien gekennzeichnet. Der Hüllkörper **31'** ist in **Fig.** 6b als geschlossener kreisförmiger Körper gestaltet.

### Implantationstechnik

Für eine Implantation wird die Bindehaut des Auges in der Nähe des Limbus der Hornhaut durch limbusparallele Schnittführung eröffnet. Bevorzugter Zugangsweg sind die temporalen Quadranten der Augenhöhle. Anschließend werden die geraden Augenmuskeln dargestellt und der Musculus rectus superior und Musculus rectus inferior stumpf auf Führungsfäden aufgenommen. Der Musculus rectus lateralis wird nahe an seinem Ansatz durch Fäden angeschlungen und vorübergehend vom Ansatz abgetrennt. Das Zielgebiet für die Implantation des Elektrodenträgers kann bestimmt werden, indem ein Markierungsinstrument außen auf der Sklera eingeführt und dessen Position durch Ophthalmoskopie festgestellt wird. Für die Befestigung des Implantates auf der Lederhaut werden dann nicht resorbierbare Fäden intraskleral verankert. Nach Einbringen des Implantats erfolgt die Befestigung, indem die Befestigungsfäden unter Nutzung der Befestigungsösen verknotet werden. Anschließend wird der Musculus rectus lateralis wieder an seinem Ansatz reinseriert und durch Nähte gesichert. Das Implantat wird zweischichtig durch Nahtadaptation der Tenon'schen Kapsel und der Bindehaut, die an den ursprünglichen Schnittführung wieder vernäht wird, gedeckt.

### Zeichnungen

Hierzu 6 Seiten Zeichnungen:
Fig. 1
Fig. 2 bestehend aus Fig. 2a, Fig. 2b, Fig. 2c, Fig. 2d
Fig. 3 bestehend aus Fig. 3a, Fig. 3b, Fig. 3c, Fig. 3d
Fig. 4 bestehend aus Fig. 4a, Fig. 4b, Fig. 4c, Fig. 4d, Fig. 4e
Fig. 5 bestehend aus Fig. 5a, Fig. 5b, Fig. 5c
Fig. 6 bestehend aus Fig. 6a, Fig. 6b

## Patentansprüche

1. Retina-Implantat zur elektrischen Stimulation der Netzhaut, wobei das Retina-Implantat eine interne Funktions-einheit umfasst, die aus Empfangsbauteilen, nachgeschalteten Elektronikeinheiten, Stimulationselektroden and einem Elektrodenträger besteht und ausgestaltet ist mit einer externen Funktionseinheit, die der Bildaufnahme, Bildverarbeitung und drahtlosen Übertragung von Bildinformation und Energie auf die interne Funktionseinheit dient zusammen-zuwirken, **dadurch gekennzeichnet, dass** die Stimulationselektroden angepasst sind, die die Netzhaut umgebenden Gewebeschichten (Aderhaut, Lederhaut) vollständig oder, nach einer Teilabtragung der Lederhaut, die verbliebenen Schichten von außen so zn penetrieren, dass die Oberflächen der Elektroden (1) sich in räumlicher Nähe zu den zu stimulierenden Zellen der Netzhaut intraretinal, intravitreal oder intrachorioidal befinden, wobei die Penetrationstiefe der einzelnen Elektroden sich unterscheiden kann und der Elektrodenträger (2) sich außerhalb des Auges, auf der äußeren Begrenzung der Lederhaut oder in einer tieferen Lederhautschicht nach deren Teilabtragung, befindet.

2. Retina Implantat nach Anspruch 1, **dadurch gekennzeichnet, dass** die Verteilung der Stimulationselektroden auf dem Elektrodenträger nicht homogen mit gleichem Abstand voneinander, sondern in unterschiedlicher Dichte angelegt ist und/oder dass die Stimulationselektroden teilweise eine Isolierung in flächiger und/oder ringförmiger Ausprägung aufweisen.

3. Retina Implantat nach den vorhergehenden Ansprüchen, **gekennzeichnet dadurch, dass** der Elektrodenträger und/oder andere Anteile des Implantates mit Ösen versehen sind, die der Nahtbefestigung des Imlantates auf der Skleraoberfläche dienen und/oder dass penetrierende Elektroden mit Widerhaken versehen sein können, die der Befestigung in der Lederhaut dienen.

4. Retina-Implantat nach den vorhergehenden Ansprüchen, **dadurch gekennzeichnet, dass** mit einer Stimulationselektrode ein Ansprechen unterschiedlicher Zellen und Zellgruppen erreicht wird, indem bei einem Stimulationsvorgang die aktive Stimulationselektrode gegen eine oder mehrere andere Stimulationselektrode(n) und/oder Referenzelektrode(n) geschaltet werden kann, wobei diese letztere(n) Stimulations- und Referenzelektrode(n) die Funktion einer Bezugselektrode einnimmt/einnehmen und **dadurch** unterschiedlich räumlich verteilte elektrische Stimulationseffekte an einer Stimulationselektrode generiert werden und/oder indem die Stimulusparameter Impulsdauer, Impulsamplitude, Impulsform und Asymmetrie bipolarer Impulsverläufe variiert werden.

5. Retina-Implantat nach den vorhergehenden Ansprüchen, **dadurch gekennzeichnet, dass** das Implantat mehrere Elektrodenträger aufweisen kann und dass jeder Elektrodenträger in mehrere gegeneinander bewegliche Segmente untereilt sein kann, die jeweils mindestens eine Stimulationselektrode tragen und dass auch andere Teile des Implantates mehrfach angelegt sein können.

6. Retina Implantat nach den vorhergehenden Ansprüchen, **dadurch gekennzeichnet, dass** mindestens eine Referenzelektrode angebracht ist, die nicht als aktive Stimulationselektrode, sondern nur als eine mit Stimulationselektroden verschaltete Gegenelektrode Verwendung findet, wobei Kennzeichen dieser ausschließlichen Referenzelektrode die fehlende räumliche Nähe zu den Zielzellen der Netzhautstimulation ist und die Referenzelektrode(n) als der Sklera aufliegende Flächenelektrode(n) oder als penetrierende Elektrode(n) ausgelegt sein und über einen eigenen Elektrodenträger verfügen oder auf der der Sklera zugewandten Fläche eines oder mehrerer Bauelemente(s) des Retina Implants angebracht sein Kann/Können.

7. Retina-Implantat nach den vorhergehenden Ansprüchen, **dadurch gekennzeichnet, dass** mehrere Empfangsspulen angebracht sind, wobei die Empfangsspulen so ausgelegt sein Können, dass sie nur der Informationsübertragung dienen.

8. Retina-Implantat nach den vorhergehenden Ansprüchen, **dadurch gekennzeichnet, dass** der Informationstransfer von der externen Einheit zum Implantat mittels einer Lichtübertragungsstrecke erfolgt.

9. Retina-Implantat nach den vorhergehenden Ansprüchen, **dadurch gekennzeichnet, dass** andere als die der Bildwahrnehmung dienenden Informationen auf das Implantat übertragen werden, beispielsweise Informationen zur tageszeitlichen Lichtperiodik dem Tagesrhythmus, oder andere durch Sensoren oder Messgeräte erfasste codierte Parameter.

10. Retina-Implantat nach vorhergehenden Ansprüchen, **dadurch gekennzeichnet, dass** die Stimulationseffekte nicht primär darauf abzielen, Phosphene auszulösen, sondern den Funktionszustand der Netzhaut, d.h. den Adaptationszustand bzw., neuronale Schwellenzustände, zu verändern oder durch die Stimulation neurotrophe Effekte zu bewirken, durch die die Progression degenerativer Prozesse der Netzhaut oder sonstiger pathologischer Veränderungen verlangsamt oder unterbunden werden.

## Claims

1. A retinal implant for electrical stimulation of the retina comprising an internal functional unit and being provided to interact with an external functional unit which is used for image capture, image processing, and wireless transmission of image information and energy to the internal functional unit, which consists of receiving components, downstream electronics units, stimulation electrodes, and an electrode support carrier, **characterised in that** the stimulation electrodes are adapted to penetrate the tissue layers (choroid, sclera) surrounding the retina either completely or, after partial removal of the sclera, to penetrate the remaining layers from the outside such that the surfaces of the electrodes (1) are situated in close spatial proximity to the retinal cells that are to be stimulated in intraretinal, intravitreal or intrachoroidal position, whereby the penetration depth of the individual electrodes can differ and the electrode support carrier (2) is situated outside the eye on the outer surface of the sclera or in a deeper layer of the sclera after partial removal of the sclera.

2. Retinal implant according to claim 1, **characterised in that** the distribution of the stimulation electrodes on the electrode support carrier is not homogenous with equal distances from each other, but with variant density and/or that the stimulation electrodes, to some extent, have an insulation designed to form coherent areas and/or to be ringshaped.

3. Retinal implant according to any one of the preceding claims **characterised in that** the electrode support carrier and/or other components of the implant are equipped with eyelets which are used to mechanically fix the implant on the scleral surface and/or **in that** the penetrating electrodes may be equipped with barbs for fixation in the sclera.

4. Retinal implant according to any one of the preceding claims, **characterised in that** responses in different cells and cell groups are obtained with a stimulation electrode by electrically connecting the active stimulation electrode to one or more other stimulation electrode(s) and/or reference electrode(s), whereby said stimulation electrode(s) and/or reference electrode(s) assume the function of a reference electrode, which generates electrical stimulation effects with variant spatial distribution on a stimulation electrode and/or responses in different cells and cell groups are obtained by variation of stimulus parameters like pulse duration, pulse amplitude, pulse shape, and asymmetry of bipolar pulse progression.

5. Retinal implant according to any one of the preceding claims, **characterised in that** the implant may be provided with multiple electrode support carriers and **in that** each electrode support carrier may be subdivided into multiple segments which can be moved with respect to each other and each carry at least one stimulation electrode, and **in that** multiples of other components of the implant may also be present.

6. Retinal implant according to any one of the preceding claims, **characterised in that** it has at least one reference electrode attached to it, which reference electrode is not an active stimulation electrode, but is used only as a counter-electrode that is electrically connected to stimulation electrodes, whereby said exclusive reference electrode is **characterised in that** is lacks close spatial proximity to the target cells of retinal stimulation, and whereby the reference electrode(s) can be designed as surface electrode(s) that is/are placed on the sclera or as penetrating electrode(s) and have a separate electrode support carrier or can be situated on the surface, which faces the sclera, of one or more component(s) of the retinal implant.

7. Retinal implant according to any one of the preceding claims, **characterised in that** it has multiple receiving coils attached to it, whereby the receiving coils can be designed such that they only serve for the transfer of information.

8. Retinal implant according to any one of the preceding claims, **characterised in that** the information transfer from the external unit to the implant proceeds through a light transmission pathway.

9. Retinal implant according to any one of the preceding claims, **characterised in that** other information than that which is needed for the perception of images, for example information about the periodic diurnal changes of light, diurnal rhythm or other encoded parameters that are detected by sensors or measuring instruments.

10. Retinal implant according to any one of the preceding claims, **characterised in that** the stimulation effects are primarily not intended to elicit phosphenes but to change the functional status of the retina, i.e. the adaptation status and/or the neuronal threshold status, or to cause neurotrophic effects through the stimulation which slow or prevent the progression of degenerative processes of the retina or other pathological changes.

## Revendications

1. Implant rétinien pour la stimulation électrique de la rétine, dans lequel l'implant rétinien comprend un module de fonctionnement interne et est réalisé pour coopérer avec un module de fonctionnement externe qui sert à l'enregistrement d'images, au traitement d'images et à la transmission sans fil d'informations sur les images et d'énergie au module de fonctionnement interne, qui se compose de composants de réception, de modules électroniques montés en aval, d'électrodes de stimulation et d'un support d'électrodes, **caractérisé en ce que** les électrodes de stimulation sont adaptées à pénétrer de l'extérieur les couches de tissu entourant la rétine (choroïde, sclérotique) entièrement ou, après une ablation partielle de la sclérotique, les couches restantes, de sorte que les surfaces des électrodes (1) se trouvent à proximité spatiale des cellules de la rétine à stimuler par voie intrarétinienne, intravitréenne ou intrachoroïdale, la profondeur de pénétration des électrodes individuelles pouvant varier et le support d'électrodes (2) se trouvant en dehors de l'oeil sur la limite extérieure de la sclérotique ou dans une couche de sclérotique plus profonde après son ablation partielle.

2. Implant rétinien selon la revendication 1, **caractérisé en ce que** la répartition des électrodes de stimulation sur le support d'électrodes n'est pas établie de manière homogène à écart identique les unes des autres, mais à densité différente et/ou que les électrodes de stimulation présentent en partie une isolation sous forme plane et/ou annulaire.

3. Implant rétinien selon les revendications précédentes, **caractérisé en ce que** le support d'électrodes et/ou d'autres portions de l'implant sont pourvus d'oeillets qui servent à la fixation par couture de l'implant sur la surface de la sclère et/ou que des électrodes pénétrantes peuvent être pourvues de barbes qui servent à la fixation dans la sclérotique.

4. Implant rétinien selon les revendications précédentes, **caractérisé en ce qu'**une réponse de différentes cellules et différents groupes cellulaires est obtenue avec une électrode de stimulation **en ce que** l'électrode de stimulation active peut être commutée contre une ou plusieurs autre(s) électrode(s) de stimulation et/ou électrode(s) de référence lors d'un processus de stimulation, cette/ces dernière(s) électrode(s) de stimulation et de référence prenant la fonction d'une électrode de comparaison et des effets de stimulation électriques à répartition spatiale différente étant ainsi générés sur une électrode de stimulation et/ou en faisant varier les paramètres de stimulus que sont la durée d'impulsion, l'amplitude d'impulsion, la forme d'impulsion et l'asymétrie de courbes d'impulsion bipolaires.

5. Implant rétinien selon les revendications précédentes, **caractérisé en ce que** l'implant peut présenter plusieurs supports d'électrodes et que chaque support d'électrodes peut être divisé en plusieurs segments mobiles les uns contre les autres qui portent chacun au moins une électrode de stimulation et que d'autres parties de l'implant peuvent également être réalisées de manière multiple.

6. Implant rétinien selon les revendications précédentes, **caractérisé en ce qu'**au moins une électrode de référence est montée, laquelle n'est pas utilisée comme électrode de stimulation active, mais uniquement comme électrode conjuguée pourvue d'électrodes de stimulation, une caractéristique de cette électrode de référence exclusive étant la proximité spatiale manquante par rapport aux cellules cibles de la stimulation de la rétine et la/les électrode(s) de référence pouvant être conçue(s) comme électrode(s) de surface reposant sur la sclère ou comme électrode(s) pénétrante(s) et pouvant disposer de son/leur propre support d'électrodes ou pouvant être appliquée(s) sur la surface tournée vers la sclère d'un ou de plusieurs constituant(s) de l'implant rétinien.

7. Implant rétinien selon les revendications précédentes, **caractérisé en ce que** plusieurs bobines de réception sont montées, les bobines de réception pouvant être conçues de sorte qu'elles ne servent qu'à la transmission d'informations.

8. Implant rétinien selon les revendications précédentes, **caractérisé en ce que** le transfert d'informations du module externe vers l'implant s'effectue au moyen d'une voie de transmission lumineuse.

9. Implant rétinien selon les revendications précédentes, **caractérisé en ce que** d'autres informations que celles servant à la perception d'images sont transmises à l'implant, par exemple, des informations concernant le photopériodisme diurne, le rythme circadien ou d'autres paramètres codés saisis par des capteurs ou appareils de mesure.

10. Implant rétinien selon les revendications précédentes, **caractérisé en ce que** les effets de stimulation n'ont pas pour but principal de dissoudre des phosphènes, mais de modifier l'état fonctionnel de la rétine, c'est-à-dire l'état d'adaptation, ou les états de seuil neuronaux, ou d'entraîner des effets neurotrophes par la stimulation grâce auxquels la progression de processus dégénératifs de la rétine ou d'autres modifications pathologiques est ralentie ou supprimée.
